# DEMANDE DE BREVET EUROPEEN

(11) **EP 1 001 033 A2**
(43) Date de publication de la demande: **17.05.2000**
(21) Numéro de dépôt: 99402666.4
(22) Date de dépôt: 26.10.1999
(51) Int. Cl.: C12P 7/56, C12N 9/28, A23L 1/03, A21D 8/04, A61K 38/47, C12R 1/245, C12R 1/25

(54) **Procédé de production de farines lactiques et produits obtenus**

(30) Priorité: 30.10.1998 FR 9813685
(71) Demandeur: Institut de Recherche pour le Développement ( IRD), 75480 Paris Cedex 10 (FR)
(72) Inventeur: Raimbault, Maurice, 34980 St. Clement de Riviere (FR); Pintado Valverde, José, 34000 Montpellier (FR); Guyot, Jean-Pierre, 34000 Montpellier (FR)
(74) Mandataire: Peaucelle, Chantal

(57) **Abrégé**

L'invention concerne un procédé de production de farines lactiques, à partir de bactéries lactiques, comprenant la fermentation d'une suspension aqueuse concentrée de substrats amylacés à l'aide de bactéries lactiques amylolytiques, le milieu de fermentation renfermant au moins 175 g/l de matière sèche en suspension dont au moins 75 g/l d'un inducteur de biosynthèse d'α-amylase et au moins 100 g/l de source protéique, à un pH de 4 à 6,5 et la récupération de la biomasse fermentée par séchage ou lyophilisation, suivie le cas échéant d'un fractionnement.

Applications des farines lactiques pour fermenter ou élaborer des compléments alimentés.

## Description

L'invention a pour objet un procédé de production de farines lactiques et les produits ainsi obtenus. Elle vise également les applications de ces produits, en particulier dans le domaine alimentaire.

Par "farines lactiques", on entend, dans la description et les revendications, des fermentats ou biomasses résultant de la culture de bactéries lactiques.

On sait que la majorité des études sur les bactéries lactiques a concerné le secteur laitier, dépourvu donc d'amidon mais riche en lactose. Quelques bactéries ont été décrites au niveau taxonomique comme pouvant dégrader l'amidon. On citera *Lactobacillus amylophillus, Lactobacillus amylovorus* ou encore *Lactobacillus brevis.* Il s'agit cependant de souches très peu productrices d'enzymes, de croissance lente ou aléatoire.

De travaux antérieurs des inventeurs ont en revanche conduit à l'isolement de bactéries lactiques capables de dégrader l'amidon de façon très efficace. En particulier, *Lactobacillus plantarum* A6, ci-après LbA6 en abrégé, a été isolée en 1988 de manioc fermenté en Afrique (Giraud *et al.,* Appl. Microbiol Biotechnol (1991) 36:379-383. Il s'agit d'une souche capable de croître sur amidon et sur glycogène avec des performances proches de celles obtenues sur glucose. Il a été démontré que, dans des conditions de contrôle de pH, il n'est pas nécessaire de gélatiniser l'amidon.

De telles bactéries lactiques amylolytiques présentent alors l'intérêt de produire des α-amylases assurant une dégradation de l'amidon cru dans des procédés naturels, de tolérer de faibles pH de l'ordre de 4,5 à 5,5, d'être thermotolérantes, leur température optimale étant entre 50 et 60°C, et d'être thermolabiles à partir de 70°C.

En simplifiant les conditions de culture classiquement rapportées pour ces bactéries, les inventeurs ont constaté qu'ils pouvaient disposer de farines directement utilisables pour des applications alimentaires.

L'invention a donc pour but de fournir un procédé de production de farines lactiques de mise en oeuvre aisée simple, économique et écologique, réalisable en continu. Elle a également pour but de fournir des farines lactiques à haute valeur ajoutée, ainsi que les ingrédients, en particulier les enzymes qu'elles renferment. Selon un autre aspect, l'invention vise la mise à profit des propriétés avantageuses des farines lactiques et de leurs constituants en particulier dans le domaine alimentaire et pharmaceutique.

Le procédé de production de farines lactiques selon l'invention est caractérisé en ce qu'il comprend
- la fermentation d'une suspension aqueuse de substrats amylacés avec des bactéries lactiques amylolytiques, le milieu de fermentation renfermant au moins 175 g/l de matière sèche en suspension, dont au moins 75 g/l d'un inducteur de biosynthèse d'α-amylase, et au moins 100 g/l de substrat amylacé, à un pH de 4 à 6,5,
- la récupération de la biomasse fermentée pour obtenir la farine lactique brute, suivie le cas échéant d'un fractionnement.

On notera l'intérêt de ce procédé dans lequel on utilise un milieu de culture très simplifié, dépourvu en particulier de sels minéraux additionnels, comme les phosphates, sulfates, chlorures, sels ammoniacaux.

De manière avantageuse, les bactéries lactiques amylolytiques mises en oeuvre sont, comme indiqué ci-dessus, thermolabiles et acidotolérantes.

Il n'est donc pas nécessaire d'effectuer un pré-traitement thermique et une stérilisation du milieu de culture. Leur capacité amylolytique leur permet de transformer l'amidon cru non gélatinisé en acide lactique sans addition d'α-amylase exogène, ni de pré-hydrolyse chimique ou thermique. Des bactéries d'intérêt pour la mise en oeuvre du procédé, assurant des bioconversions avec des rendements élevés pouvant atteindre 90 à 95%, comprennent Lb A6 et des bactéries productrices de L(+) lactate, notamment de l'espèce *Lactobacillus paracasei paracasei,* en particulier de souches de ce type résistantes à la nisine. On citera spécialement la souche de Lact*obacillus paracasei paracasei,* désignée ci-après par R10107, déposée le 27 mai 1998 à la CNCM (Collection Nationale de Culture de Microorganismes) à l'Institut Pasteur sous le n° I-2030. Cette souche est nouvelle et, en tant que telle, entre dans le champ de l'invention. Elle provient de bac de rejet de sous-produits et d'effluents de manioc fermenté.

La position taxonomique de R10107 a été confirmée par la technique moléculaire de l'ARN 16S. Le séquençage d'une partie de son ARN, comparé aux banques de données de gènes, a confirmé son appartenance à l'espèce *Lactobacillus paracasei paracasei,* étant donné sa très forte homologie avec la séquence de l'ARN 16S de la souche type décrite.

Une étude physiologique du milieu MRS-amidon et MRS-glycogène montre qu'elle est capable de dégrader et assimiler l'amidon et le glycogène, avec une biosynthèse élevée d'α-amylase et des rendements de transformation en acide lactique voisins de ceux obtenus avec le glucose, à savoir de plus de 98%.

De manière avantageuse, il s'agit d'une souche homolactique, produisant pratiquement exclusivement la forme L(+) de l'acide lactique, à savoir celle assimilable par l'organisme humain. Par le test Bohringer, on mesure une production de 94,1% d'acide L(+) lactique contre 5,8% d'acide D-lactique.

Les inventeurs ont également mis en évidence la forte résistance à la nisine de R10107.

Dans les études réalisées, on observe ainsi une zone d'inhibition pour une concentration de 1 g/l de nisine, inférieure à 1 mm.

Cette résistance à la nisine a également été observée avec Lb A6, avec une zone d'inhibition de 10 à 12 mm.

Cette propriété de résistance à la nisine des souches bactériennes, qui s'ajoute à leurs autres propriétés avantageuses, constitue un caractère de grand intérêt, étant donné que la nisine est une substance bactéricide à très large spectre, active non seulement contre les Gram+ mais aussi contre les Gram-, et en particulier de nombreux pathogènes de l'intestin. Il s'agit notamment du seul "antibiotique" qui soit autorisé comme additif dans les aliments.

Dans une variante de réalisation de l'invention, lesdites bactéries lactiques amylolytiques résistantes à la nisine sont mises en co-culture avec des souches productrices de nisine.

Les bactéries utilisées selon l'invention sont mises en oeuvre à des concentrations de 10⁶ et 10⁷/ml.

Les inducteurs de synthèse d'α-amylase par les bactéries sont avantageusement des produits amylacés, comme le manioc ou les farines de céréales, ou des sources de carbone, comme le cellobiose ou le glycogène. Comme source protéique, on a avantageusement recours à des produits tels que la farine ou le lait de soja, ou des hydrolysats protéiques, par exemple le corn steep liqueur.

De manière avantageuse, la durée de fermentation peut être courte, notamment de l'ordre de 12 à 24 heures.

La biomasse formée est récupérée et constitue la totalité du milieu de culture. Elle peut être simplement déshydratée en vue d'applications ultérieures, sans nécessité de post-traitements d'extraction complexes et coûteux, ni séparation de la biomasse du milieu de culture et élimination de sels chimiques. On notera avec intérêt le caractère écologique de cette technologie qui ne génère pas d'effluents.

Un ou plusieurs des éléments constitutifs de la farine fermentée brute peuvent être sélectivement séparés, si on le souhaite, au cours d'une étape ultérieure.

Il est ainsi possible d'isoler l'α-amylase produite sous forme concentrée ainsi que l'acide lactique.

L'α-amylase est séparée par exemple de la biomasse par ultrafiltration et peut être alors purifiée et formulée selon les applications envisagées.

La purification comprend par exemple un traitement de précipitation au sulfate d'ammonium, d'adsorption et d'élution sur support de chromatographie.

De même l'acide lactique produit par fermentation des bactéries mises en oeuvre peut être isolé de la biomasse, en opérant comme ci-dessus par exemple par ultrafiltration. Le produit est alors purifié en vue d'applications ultérieures.

Une valorisation supplémentaire est apportée au procédé de l'invention lorsqu'on récupère les bactéries à partir de la biomasse.

L'invention vise également, en tant que produits nouveaux, les farines lactiques brutes obtenues.

Ces farines sont caractérisées par une forte concentration en α-amylase et en bactéries.

Ainsi, leur concentration en enzyme est supérieure à 50 Unités d'enzymes/g, et peut même aisément atteindre 70 ou 80 Unités/g. Leur concentration en bactéries est supérieure à 10¹⁰ cellules viables/g de farine et atteint généralement 1,5.10¹⁰ cellules viables/g de farine.

Les analyses microbiologiques montrent que dans les conditions de culture non stériles, les contaminants naturels des farines lactiques ne se développent pas et ont même une forte tendance à diminuer, voire à disparaître.

Selon un aspect de grand intérêt, les propriétés rhéologiques de ces farines sont telles qu'elles permettent de fabriquer des aliments à plus forte densité énergétique pour une même viscosité, grâce à la présence à la fois d'α-amylase et d'acide lactique dans la farine lactique.

De plus, selon un aspect avantageux de l'invention, les bactéries lactiques mises en culture sont des germes GRAS (Generally Recognized As Safe). Elles sont donc particulièrement appropriées pour des usages en alimentation.

Les farines de l'invention sont ainsi directement utilisables comme additifs en panification, pour favoriser le développement de la levure de boulangerie lors du levage de la pâte, et provoquer dans le même temps l'acidification lactique qui permet d'obtenir la caractéristique acidulée du pain au levain naturel. Elles sont ainsi utilisables comme additifs pour la fabrication de pain, pizza, biscuit ou patisserie.

Leur utilisation permet aussi une plus longue conservation grâce à l'acide lactique qu'elles contiennent qui freine la recontamination par des microflores bactériennes ou fongiques.

Ces farines sont également utilisables en malterie pour favoriser le développement de la levure de bière et renforcer la teneur en amylase du malt.

D'une manière générale, les farines lactiques sont utilisables comme additifs probiotiques dans des préparations de type alimentaire, diététique ou pharmaceutique.

Les enzymes purifiées isolées selon l'invention sont utilisables, en panification classique de pain doux, sans production d'acide lactique, grâce à l'action de l'enzyme sur l'amidon cru de la farine de blé, ce qui permettra un développement plus rapide et plus intense de la levure pour la levée de la pâte. Une autre utilisation des enzymes concernent les applications pharmaceutiques par exemple comme aide digestive, dans des cas de carence de fabrication de sucs digestifs, ou pour une meilleure digestion d'amidons crus, mal cuits, rétrogradés ou peu digestes. Ces α-amylases peuvent également favoriser l'assimilation du glycogène.

Les farines brutes selon l'invention ainsi que les α-amylases produites peuvent être également utilisées comme additifs dans des soupes, plats cuisinés ou crèmes glacées.

D'autres caractéristiques et avantages de l'invention sont donnés dans les exemples qui suivent.

### Exemple 1: Production de farines lactiques par fermentation de Lb A6.

- farine F1
   On utilise un milieu de culture composé uniquement de farine de manioc, en tant que produit amylacé, à raison de 300g/l, et de farine de soja, comme source protéique, à raison de 100g/l.
   Le pH du milieu est de 5,75. On ajoute 100 ml d'inoculum de Lb A6 issu de 24 h d'étuve. Le pH est alors de 5,18 et est ensuite régulé par ajout de soude 2N.
   La fermentation est réalisée à 30°C. Après 2h de fermentation, le pH est diminué à 5, puis après 22h de fermentation, est inférieur à 4.
   Après 24h de fermentation, on récupère le milieu de culture, on le lyophilise et on réduit en poudre par broyage. On obtient 378, 56g de farine brute.
- farine F2
   On opère comme précédemment, mais on régule le pH à 5,5 par ajout de chaux 2N. On récupère 385,5g de farine lyophilisée.
- farine F3
   On opère comme pour la farine F1, mais le fermentat récupéré est séché sous hotte ventilée pendant 48h, puis broyé à l'aide d'un robot électrique.
   Comme témoin, on utilise un milieu classique renfermant 150g de farine de manioc et 500ml de lait de soja. On congèle ensuite le fermentat, puis on le lyophilise.
   Les farines brutes sont soumises à une analyse HPLC.

### Les échantillons sont dilués au 6ème.

On a applique le protocole suivant :
On utilise 2,5 g de farine lyophilisée dans 50 ml d'eau. Cette suspension est soumise à agitation puis acidifiée à l'aide de HCl 11N jusqu'à pH 2 et à nouveau soumise à agitation. On prélève 1,5 ml qu'on distribue dans des eppendorfs. On procède à une centrifugation pendant 10 min. à 10 000 rpm. On prélève 1 ml du surnageant, qu'on répartit dans des eppendorfs. Les produits sont congelés lorsque l'analyse ne peut être effectuée immédiatement. On procède ensuite à une filtration avec des filtres de 0,45 µm, puis on injecte les produits dans la colonne HPLC.

Les résultats donnés ci-après sont exprimés en pourcentage d'acide lactique par gramme de farine :
échantillon témoin 1%
F1 : 8,9 %
F2 : 8,35%
F3 : 8,01%

L'étude de l'effet du type de fermentation sur la relation viscosité/concentration dans les bouillies donne les résultats suivants :

Les farines F1, F2, F3 permettent de préparer pour une même viscosité des bouillies ayant des concentrations de 20 à 50 % plus élevées qu'avec les témoins.

On dispose ainsi de bouillies fortement concentrées en α-amylases, de plus forte valeur énergétique.

Les fermentations effectuées conformément à l'invention permettent donc une amélioration substantielle de la densité énergétique des bouillies. On rapporte dans le tableau suivant la composition biochimique de farine fermentée Lb A6 et séchée au rotavapor.

| | F1 | F2 | F3 | F4 | F5 | F6 | F8 |
|---|---|---|---|---|---|---|---|
| | 75/100 | 75/100 | 100/100 | 100/100 | 200/100 | 200/100 | 300/100 |
| % Humidité | | | | | | | |
| | 4,11 | 7,08 | 10,11 | 9,16 | 5,03 | | 7,76 |
| % Protéines | | | | | | | |
| | 14,01 | 15,25 | 10,49 | 17,94 | 13,65 | 14,67 | 10,43 |
| % Sucres | | | | | | | |
| | 3,39 | 4,05 | 3,22 | 3,45 | 3,74 | 3,29 | 3,34 |
| % Amidon | | | | | | | |
| | 35,42 | 24,43 | 30,89 | 28,6 | 54,89 | 32,4 | 35,59 |
| % Lipides | | | | | | | |
| | 14,06 | 9,8 | 10,97 | 9,37 | 6,68 | 6,76 | 5,62 |
| % Fibres | | | | | | | |
| | 2,17 | 2,58 | 2,58 | 2,14 | 3,1 | 2,1 | 2,52 |
| pH | | | | | | | |
| | 5,55 | 5,29 | 5,04 | 5 | 5,08 | 5 | 5,13 |
| Ac. Lactique | | | | | | | |
| % | 13,28 | 13,5 | 16 | 13,55 | 11,16 | 13,51 | 13,77 |
| | | | | | | | |
| Amylase | | | | | | | |
| (UE/L) | 4565,3 | 4220,81 | 7552,07 | 6991,61 | 4491,25 | 4893,25 | 7200,66 |

Les comptages microbiologiques à 0 h et 24 h de fermentation lactique montrent clairement l'effet de dominance de la souche inoculée dans un environnement non aseptique qui permet même de réduire de façon très notable les taux de contamination de farines, ce qui représente un avantage décisif pour la fabrication industrielle de farines naturelles non traitées, tout en assurant une sécurité de fermentation lactique contrôlée.

### EXEMPLE 2 : Production de farine par fermentation de bactéries lactiques

On utilise comme milieu de culture 50 ml de lait de soja et 15 g de farine soit de manioc (a), soit de blé (b) dans un erlen de 100 ml.

On inocule avec 4 ml de Lb A6 ou R10107.

On lyophilise les fermentats récupérés et on mesure la perte de poids :
blé : Pds ᵢ = 67 Pds _{f}=58,98g Perte de poids = 11,97%
manioc : Pds ᵢ =67 g Pds _{f}=59,89g Perte de poids=10,61%
Les poids secs sont réalisés sur les échantillons lyophilisés :
Teneur en eau du blé : 3,7%
Teneur en eau de manioc : 2,86%

| **pH** | | |
|---|---|---|
| | **A6** | **R10107** |
| **Manioc 0 h** | 5,35 | 5,27 |
| **Manioc 24 h** | 3,66 | 3,70 |
| **Blé 0 h** | 5,20 | 5,29 |
| **Blé 24 h** | 3,34 | 3,40 |

| **Souches** | **Acidités (N)** |
|---|---|
| Blé A6 | 0,0034 |
| Blé R10107 | 0,0031 |
| Manioc A6 | 0,0022 |
| Manioc R10107 | 0,0026 |

### EXEMPLE 3 : Procédé d'obtention d'acide L+ lactique

On utilise une suspension amylacée concentrée, sans traitement thermique, de 300g/l de farine d'amidon cru et de 100g/l de farine de soja. On ajoute un inoculum de 10% (v/v) d'une préculture de R10107 qui permet d'obtenir 3 x 10⁶ UFC/ml dans le milieu inoculé au temps initial. On réalise le procédé en continu en régulant le pH à 5,5 en utilisant de carbonate de calcium comme agent neutralisant qui provoque la précipitation du lactate de calcium à forte concentration d'acide lactique dans le milieu.

Après 24 h de culture, on obtient une concentration supérieure à 30g/l d'acide lactique, dont 94,1% sous forme L+lactique et une concentration cellulaire de 2,3 x 10¹⁰ UFC/ml, soit une biomasse de 10 000 fois supérieure, avec un taux de croissance de 1,45 h⁻¹.

Une double ultrafiltration, sur membranes calibrées, permet de récupérer une première fraction contenant l'acide L(+) lactique concentré, puis l'α-amylase dans une seconde fraction concentrée. Le rétentat contient une forte concentration cellulaire de cette bactérie aux propriétés probiotiques pour d'autres usages alimentaires. Ceci permet d'obtenir une triple valorisation du procédé.

### EXEMPLE 4 : Production de farines lactiques à l'aide de co-cultures

On réalise directement une co-culture dans le milieu de culture très simplifié de l'exemple 3, inoculé non seulement par la souche R10107 amylolytique, mais également par la souche *Lactococcus lactis* ATCC 14454, pour obtenir une concentration initiale de 3. 10⁷ UFC/ml (concentration dix fois supérieure à celle de Lb *paracasei* R10107). Cette co-culture directe sur milieu de culture très simple à base d'amidon, résultat d'une relation symbiotique entre la souche amylolytique qui fournit la source de carbone assimilable, et la souche productrice de nisine qui protège des contaminations éventuelles en milieu non stérile, permet d'obtenir en moins de 48 heures un milieu de culture qui contient les deux microflores vivantes à haute concentration (environ 10⁹ UFC/ml de chaque bactérie lactique) à fort pouvoir amylolytique, contenant 60 g/l d'acide L-lactique et surtout une concentration minimale de 0,5 à 1 g/l de nisine. L'obtention de nisine par cette voie correspond à une fabrication de faible coût.

Par séchage de ce milieu de culture, on obtient une farine probiotique qui possède un pouvoir antibiotique autorisé pour l'alimentation humaine ou animale, de bonne conservation et pouvant donc servir d'ingrédient alimentaire compte tenu de ses bonne propriétés d'acceptabilité alimentaire.

L'invention fournit donc une technique de fermentation simplifiée dans laquelle, de manière originale, on n'utilise aucun traitement thermique que ce soit pour gélatiniser l'amidon, pasteuriser ou stériliser le milieu. La fermentation lactique se déroule ainsi en présence de la microflore naturellement présente dans les farines et eaux utilisées, grâce à l'inoculation des souches lactiques amylolytiques mises en oeuvre.

## Revendications

1. Procédé de production de farines lactiques, à partir de bactéries lactiques, caractérisé en ce qu'il comprend
- la fermentation d'une suspension aqueuse de produits amylacés avec des bactéries lactiques amylolytiques, le milieu de fermentation renfermant au moins 175 g/l de matière sèche en suspension, dont au moins 75 g/l d'un inducteur de biosynthèse d'α-amylase, et au moins 100 g/l de produit amylacé, à un pH de 4 à 6,5,
- la récupération de la biomasse fermentée pour obtenir la farine lactique brute, suivie le cas échéant d'un fractionnement.

2. Procédé selon la revendication 1 caractérisé en ce qu'on utilise des bactéries lactiques amylolytiques thermolabiles, acidotolérantes, capables de dégrader l'amidon cru non gélatinisé.

3. Procédé selon la revendication 2, caractérisé en ce qu'on utilise *Lactobacillus plantarum* A6.

4. Procédé selon la revendication 2, caractérisé en ce qu'on utilise des bactéries lactiques, amylolytiques, productrices de L(+) lactate, en particulier résistantes à la nisine.

5. Procédé selon la revendication 4, caractérisé en ce qu'on utilise une souche bactérienne de l'espèce *Lactobacillus paracasei paracasei.*

6. Procédé selon la revendication 5, caractérisé en ce qu'on utilise la souche R10107 déposée le 27 mai 1998 à la CNCM sous le n° I-2030.

7. Procédé selon l'une quelconque des revendications 1 à 6, caractérisé en ce qu'on met en oeuvre les bactéries à des concentrations de 10⁶ à 10⁷ UCF/ml.

8. Procédé selon l'une quelconque des revendications 1 à 7, caractérisé en ce qu'on réalise une co-culture avec des souches bactériennes productrices de nisine.

9. Procédé selon l'une quelconque des revendications 1 à 8, caractérisé en ce que le milieu de fermentation renferme au moins 400 g/l de matière sèche en suspension.

10. Procédé selon l'une quelconque des revendications 1 à 9, caractérisé en ce qu'on utilise, comme inducteur de synthèse d'α-amylase, des produits amylacés comme des farines de céréales, ou des sources de carbone, comme le cellobiose ou le glycogène.

11. Procédé selon l'une quelconque des revendications 1 à 10, caractérisé en ce que les produits amylacés ne sont pas soumis à un traitement thermique et ne sont additionnés de sels minéraux.

12. Procédé selon l'une quelconque des revendications 1 à 11, caractérisé en ce qu'on utilise, comme produits amylacés, des produits tels que la farine ou le lait de soja.

13. Procédé selon l'une quelconque des revendications 1 à 12, caractérisé en ce que la biomasse fermentée récupérée est séchée et lyophilisée.

14. Procédé selon la revendication 13, caractérisé en ce qu'on sépare au préalable, de la farine brute, l'α-amylase produite sous forme concentrée et/ou l'acide lactique et/ou les bactéries.

15. Farines lactiques, caractérisées par une concentration en α-amylase supérieure à 50 Unités d'enzymes/g, notamment de 70 ou 80 Unités/g et une concentration en bactéries supérieure à 10¹⁰ cellules viables par g de farine, notamment de l'ordre de 1,5.10¹⁰ cellules viables par g de farine.

16. α-amylases telles qu'obtenues selon le procédé de la revendication 14, sous forme appropriée à leurs applications dans le domaine alimentaire, diététique, ou pharmaceutique.

17. Souche bactérienne de l'espèce *Lactobacillus paracasei paracasei,* R10107, déposée le 27 mai 1998 à la CNCM sous le n°I-2030.

18. Applications des farines lactiques selon la revendication 15 et de leurs composants, notamment des α-amylases selon la revendication 16, des bactéries lactiques amylolytiques et de l'acide L(+) lactique, pour l'élaboration de compléments alimentaires probiotiques ou diététiques.

19. Utilisation des farines lactiques selon la revendication 15 et/ou des α-amylases selon la revendication 16, en panification.

20. Utilisation des farines lactiques selon la revendication 15 et/ou des α-amylases selon la revendication 16, en malterie.

21. Utilisation des a-amylases selon la revendication 16, pour la fabrication de médicaments utilisables comme aide digestive ou pour favoriser l'assimilation du glycogène.
